# EUROPÄISCHE PATENTANMELDUNG

(11) **EP 1 293 223 A1**
(43) Veröffentlichungstag der Anmeldung: **19.03.2003**
(21) Anmeldenummer: 02020199.2
(22) Anmeldetag: 10.09.2002
(51) Int. Cl.: A61M 5/30

(54) **Behälter zur nadellosen Injektion von medizinisch wirksamen Substanzen**

(30) Priorität: 12.09.2001 DE 10144794
(71) Anmelder: Schott Glas, 55122 Mainz (DE); Carl-Zeiss-Stiftung trading as Schott Glas, 55122 Mainz (DE)
(72) Erfinder: Fabian, Arthur, 9000 St. Gallen (CH); Kuhr, Markus, Dr., 55597 Wöllstein (DE); Holschumacher, Ralf, Dr., 55130 Mainz (DE)
(74) Vertreter: Fuchs Mehler Weiss & Fritzsche

(57) **Zusammenfassung**

Derartige Behälter sind mit einer Druckeinrichtung integriert oder integrierbar und weisen austrittsseitig einen konvergierenden Kopfteil (3) mit einer durch eine Kappe (6) verschließbaren düsenförmigen Austrittsöffnung (4) für die im Behälter eingefüllte, flüssige medizinische Substanz auf.

Die bekannten Behälter bestehen entweder aus Kunststoff oder aus Glas und erfüllen, je nach Konstruktion, nur teilweise die Anforderungen, die an solche Behälter gestellt werden, nämlich hohe Barriereeigenschaften (um ein "Austrocknen" von vorgefüllten Behältern zu vermeiden), sowie hohe und kurzzeitige Druckspitze beim Applizieren, und Bruchsicherheit.

Um diesen Anforderungen in Gänze gerecht zu werden, sieht die Erfindung vor, daß der Behälter (1') aus zwei fünktionellen Bauteilen besteht, einem Kunststoffgehäuse (10) mit einstückig angeformtem Kopfteil (3) und einem Glaszylinder (9), der in dem Kunststoffgehäuse (10) unter Bildung der Innenwand des Behälters (1') eingesetzt ist.

## Beschreibung

Die Erfindung bezieht sich auf einen Behälter zur nadellosen Injektion von medizinisch wirksamen Substanzen, der mit einer Druckeinrichtung integriert oder integrierbar ist, und der austrittsseitig einen konvergierendes Kopfteil mit einer durch eine Kappe verschließbaren düsenförmigen Austrittsöffnung für die jeweilige Substanz aufweist.

Zur subkutanen sowie intramuskulären Injektion von Arzneimitteln, einschließlich Diagnostika, werden typischerweise Spritzen mit einer Injektionsnadel verwendet, mittels der beim Injizieren die Haut des Patienten durchstochen wird. Da es zahlreiche Patienten gibt, die eine starke Abneigung gegen Injektionsnadeln haben, eine sogenannte Nadelphobie, und weil die Injektionsnadeln typischerweise mit Silikonöl beschichtet sind und daher beim Injizieren Silikonöl zum Teil in den Körper des Patienten gelangen könnte, wurden Vorrichtungen zur nadellosen Injektion von medizinisch wirksamen Substanzen entwickelt, mittels derer die zu injizierende Substanz nadellos mittels hohem Druck durch die Haut des Patienten hindurch "geschossen" und im darunter liegenden Gewebe deponiert wird.

Vorrichtungen zur nadellosen Injektion von Arzneimitteln sind bekannt. So beschreiben die WO 98/499 94 sowie die US 5 704 911 und die WO 97/278 34, entsprechende Vorrichtungen mit integrierten oder integrierbaren Behältnissen, die als Lagerungsbehältnis für Medikamente geeignet sind, in denen durch zugehörige Einrichtungen, wie ein unter Federvorspannung stehender Kolben, im Applizierfall der notwendige hohe Injizierdruck aufbringbar ist. Diese Behältnisse weisen eine düsenförmige Austrittsöffnung für die medizinisch wirksame Substanz mit einem ebenen, flächigen Rand für die Auflage auf die Haut des Patienten beim Injizieren auf. Diese Austrittsöffnung muß während der Lagerung flüssigkeits- und keimdicht verschlossen sein.

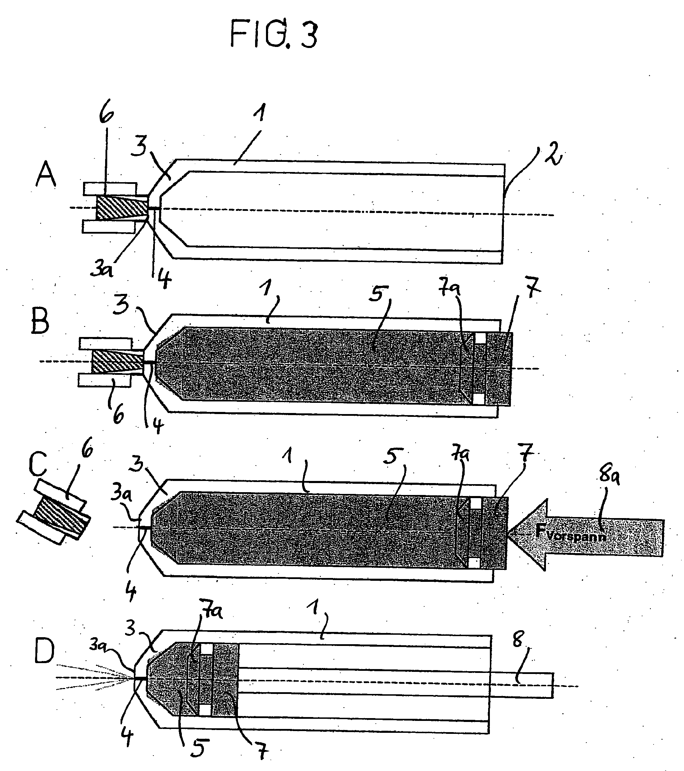

Anhand der Fig. 3 soll das Prinzip der nadellosen Injektion mit marktüblichen Behältnissen näher erläutert werden.

Diese marktüblichen Behälter 1 sind typischerweise zylindrisch ausgebildet, sind an einem Ende 2 über den ganzen Querschnitt offen und besitzen am anderen Ende einen konisch zulaufenden Kopf 3 mit einer ebenen Auflagefläche 3 a, in der mittig eine düsenförmige Öffnung 4 ausgebildet ist. Vor dem Befüllen des Behälters 1 über das offene Ende 2 mit einem Medikament 5 wird die düsenförmige Öffnung 4 mit einer Kappe 6 verschlossen (Figurenteil A). Nach dem Befüllen des Behälters 1 wird der Behälter am offenen Ende 2 mit einem Stopfen 7, typischerweise mit einem Kunststoffstopfen, verschlossen, der einen kolbenförmigen Abschnitt 7 a aufweist (Figurenteil B). Bei der Verabreichung des Medikamentes 5, subkutan oder intramuskuläre Verabreichungsform für z.B. Impfstoffe, wird nach dem Entfernen der die Düse 4 verschließenden Kappe 6 der Kolbenstopfen 7, 7 a über eine mit der Kraft 8 a vorgespannte Kolbenstange 8 axial zur Behälterform in Richtung Düse 4 "geschossen" (Figurenteile C, D). In der Behälterkammer werden dabei laut Angaben einiger Hersteller Drücke von bis zu 500 bar aufgebaut. Das Medikament 5 tritt dabei mit hoher Geschwindigkeit aus der Düse 4 aus und der Druck reicht für eine Verabreichung des Medikamentes unter die menschliche Haut.

Die bekannten Behältnisse zur nadellosen Injektion von medizinisch wirksamen Substanzen kommen typischerweise gemäß dem im Figurenteil B dargestellten Zustand vorgefüllt und zur einmaligen Verabreichung bestimmt (single-use-Behälter) auf den Markt. Die vorgefüllten Behälter, die das Medikament sozusagen bevorraten, sind entweder Glas- oder Kunststoff-Behälter. Teilweise sind auch schon Kunststoffbehälter, die aus zwei verschiedenen Kunststoffen bestehen, sogenannte 2-K-Spritzgießteile, auf dem Markt vorgestellt worden.

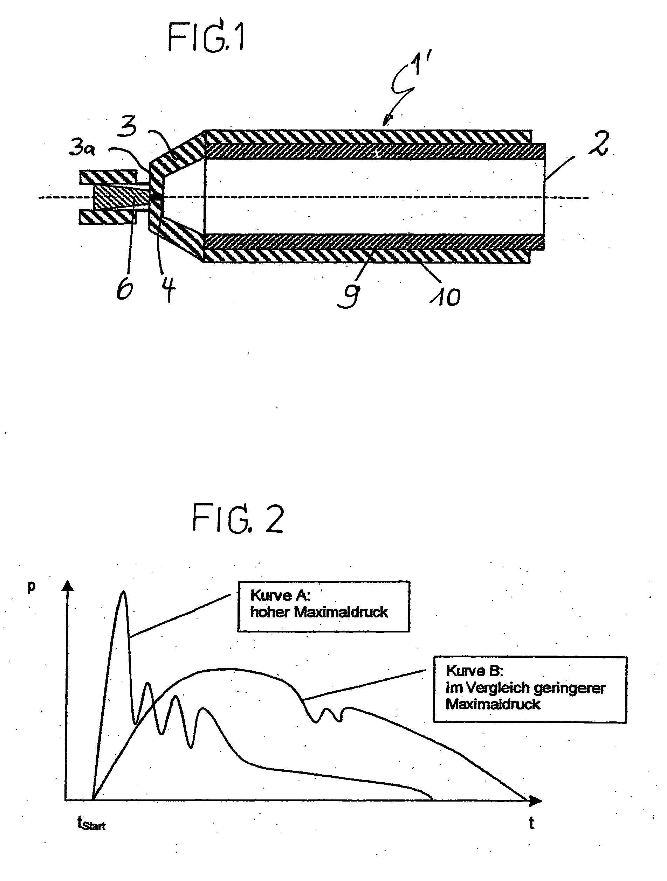

Derartige vorgefüllte Behälter müssen eine Reihe von Anforderungen erfüllen.
- Das Behältersystem (Behälter 1, Stopfen 7 und Kappe 6) muß über ausreichende Barriereeigenschaften verfügen.
- Die eingesetzten Materialien sollen möglichst γ-strahlensterilisierbar oder dampfsterilisierbar sein.
- Die Verabreichung soll möglichst schmerzfrei sein, Spuren auf der Haut nach einer Verabreichung sollen nach Möglichkeit nicht sichtbar sein. Um diese Anforderungen zu erfüllen, muß der Druckverlauf im Behälter einer definierten Kurve folgen, die in Fig. 2 dargestellt ist. Anzustreben ist die Kurve A mit einer hohen Druckspitze zu Beginn der Verschiebung des Stopfens 7 in Verbindung mit einem anschließend schnellen Druckabfall. Dies ist nur dann gegeben, wenn die Kraftübertragung der die Vorspannkraft 8 a typischerweise liefernden Feder möglichst verlustfrei in kinetische Energie umgewandelt wird. Folgende Phänomene verursachen jedoch Reibungsverluste und verhindern dadurch den hohen kurzen Druck-Peak, d.h. verursachen einen Druckverlauf mit einem geringeren, und länger anstehenden Maximaldruck, gemäß der Kurve B in Fig. 2:
   ○ hohe Haft- bzw. Gleitreibungswerte des Stopfensystems 7, 7 a
   ○ kleinste Unebenheiten auf der Behälteririnenoberfläch
   ○ Einsatz von "zu weichen" Materialien (Weichgummi als Stopfenmaterial oder ein im Verhältnis weicher Kunststoff als Behältermaterial)
- Der Bruch des Behälters bei der Applikation infolge des hohen Druckes muß unter allen Umständen ausgeschlossen werden.

Der Erfindung liegt die Aufgabe zugrunde, den eingangs bezeichneten Behälter zur nadellosen Injektion von medizinisch wirksamen Substanzen so auszubilden, daß er diesen Anforderungen genügt.

Die Lösung dieser Aufgabe gelingt bei einem Behälter zur nadellosen Injektion von medizinisch wirksamen Substanzen, der mit einer Druckeinrichtung integriert oder integrierbar ist, und der austrittsseitig ein konvergierendes Kopfteil mit einer durch eine Kappe verschließbaren düsenförmigen Austrittsöffnung für die jeweilige Substanz aufweist, gemäß der Erfindung dadurch, daß der Behälter aus zwei funktionellen Bauteilen besteht, einem Kunststoffgehäuse mit einstückig angeformten Kopfteil und einem Glaszylinder, der in dem Kunststoffgehäuse unter Bildung der Innenwand des Behälters eingesetzt ist.

Das erfindungsgemäße zweischalige Behältersystem verfügt allein über die Materialeigenschaften von Glas und Kunststoff sowie über die Gestaltung des Kunststoff-Formteiles über ausreichende Barriereigenschaften. Glas ist dampfsterilisierbar, ausgewählte Kunststoffe sind dampf- oder strahlensterilisierbar. Durch die mechanischen Eigenschaften des Glaszylinders können in Verbindung mit dem richtigen Stopfen, mit oder ohne Innensilikonisierung des Glaszylinders, niedere Haft- bzw. Gleitreibungswerte erzielt werden. Dadurch kann eine Druckkurve analog zur Kurve a in Fig. 2 erzielt werden.

Ein erfindungsgemäßer Behälter, ausgebildet als vorgefüllter, steril verpackter Behälter für einmaligen Gebrauch, läßt sich auf vorteilhaft einfache Weise schaffen, wenn gemäß einer Ausgestaltung der Behälter aus jeweils sterilem Kunststoffgehäuse mit Kopfteil und Glaszylinder sowie steriler Kappe besteht, die in einem Sterilraum montiert, befüllt und verschlossen worden sind.

Ein besonders einfacher Aufbau des Behälters ist gegeben, wenn einmal der Glas-Innenzylinder aus einem marktüblichen Type I Glasrohr mit vorgegebenen Toleranzen abgelängt ist und zum anderen das Kunststoffgehäuse mit angeformtem Kopfteil ein Spritzgießteil ist.

Vorzugsweise ist dabei neben dem Glas-Innenzylinder auch das Kunststoffgehäuse transparent ausgebildet, um den Inhalt des Behälters visuell inspizieren zu können. Als Kunststoffmaterialien werden dabei vorzugsweise verwendet:
- Cycloolefine Copolymere (COC)
- Cycloolefine Polymere (COP)
- Polycarbonate (PC)
- Polystyrol (PS)
- Acrylnitrit-Butadien-Styrol-Pfropfpolymer (ABS)
- Polymethylmethacrylat (PMMA)
- Polyethylenterephtalat (PET)

Anhand eines in den Zeichnungen dargestellten Ausführungsbeispieles wird die Erfindung näher beschrieben.

Es zeigen:
- Fig. 1: in einer schematisierten Längsschnitt-Darstellung den zweischaligen Aufbau des erfindungsgemäßen Behälters zur nadellosen Injektion von medizinisch wirksamen Substanzen,
- Fig. 2: ein Diagramm zur Darstellung der zeitlichen Druckverläufe in einem Behälter zur nadellosen Injektion bei der Applikation, und
- Fig. 3: in vier Figurenteilen A - D Aufbau, Montage und Applikation eines bekannten Behälters zur nadellosen Injektion.

In Figur 1 ist ein Ausführungsbeispiel des erfindungsgemäßen Behälters 1' zur nadellosen Injektion von medizinisch wirksamen Substanzen dargestellt. Dieser Behälter 1' besitzt, wie der bekannte Behälter nach Fig. 3, einen Kopf 3 mit einer Düse 4 im Aufsetzteil 3 a, die durch eine Kappe 6 verschlossen ist. Während in Fig. 3 die Wandung des Behälters aus einem Teil besteht und einstückig mit dem Kopf 3 verbunden ist, besteht das in Fig. 1 dargestellte erfindungsgemäße Behältersystem aus zwei funktionellen Bauteilen, die vor der Befüllung sterilisiert und im Anschluß in einem Sterilraum montiert, befüllt und verschlossen werden, nämlich einem
- Innenzylinder 9 aus Glas
- Kunststoffgehäuse 10, das integral mit dem Kopf 3 verbunden ist.

Als Innenzylinder 9 aus Glas kann ein marktübliches Type I Glasrohr eingesetzt werden. Die Abmaße Gesamtlänge sowie Innen- und Außendurchmesser sind allerdings definiert zu tolerieren.

Bei dem Kunststoffgehäuse 10 handelt es sich vorzugsweise um ein transparentes Spritzgießteil. In dem angespritzten konvergierenden Kopf 3 befindet sich die Düse 4, die von außen mit einer Kappe 6 verschlossen wird. Mögliche Kunststoffmaterialien, aus denen das Gehäuse 10 ausgeführt werden kann, sind:
- Cycloolefine Copolymere (COC)
- Cycloolefine Polymere (COP)
- Polycarbonate (PC)
- Polystyrol (PS)
- Acrylnitril-Butadien-Styrol-Pfropfpolymer (ABS)
- Polymethylmethacrylat (PMMA)
- Polyethylenterephtalat (PET)

Die Montage der Einzelteile erfolgt in steriler Umgebung (aseptisch). Die Einzelteile, d.h. das Kunststoffgehäuse 10, der Glas-Innenzylinder 9 und die Kappe 6 werden dabei in steriler Form angeliefert. Die Montage des Behälters kann in der beschriebenen Reihenfolge ausgeführt werden:

### (Montage des Behälters)

I. Kappe 6 in Kunststoffgehäuse 10 einsetzen
II. Glaszylinder 9 in Kunststoffgehäuse 10 einbringen und positionieren

### (Befüllung)

III. Befüllung des Behälters )
IV. Setzen des Stopfens ) entsprechend Fig. 3, Teile B und D
V. Aufsetzen des Spannelementes)
VI. Verpacken des Systems (nicht dargestellt)

## Patentansprüche

1. Behälter zur nadellosen Injektion von medizinisch wirksamen Substanzen, der mit einer Druckeinrichtung integriert oder integrierbar ist, und der austrittsseitig einen konvergierenden Kopfteil (3) mit einer durch eine Kappe (6) verschließbaren düsenförmigen Austrittsöffnung (4) für die jeweilige Substanz aufweist, **dadurch gekennzeichnet, daß** der Behälter (1') aus zwei funktionellen Bauteilen besteht, einem Kunststoffgehäuse (10) mit einstückig angeformtem Kopfteil (3) und einem Glaszylinder (9), der in dem Kunststoffgehäuse (10) unter Bildung der Innenwand des Behälters (1') eingesetzt ist.

2. Behälter nach Anspruch 1, ausgebildet als vorgefüllter Behälter für einmaligen Gebrauch, **dadurch gekennzeichnet, daß** der Behälter aus jeweils sterilem Kunststoffgehäuse (10) mit Kopfteil (3) und Glaszylinder (9) sowie steriler Kappe (6) besteht, die in einem Sterilraum montiert, befüllt und verschlossen worden sind.

3. Behälter nach Anspruch 1 oder 2, **dadurch gekennzeichnet, daß** der Glas-Innenzylinder (9) aus einem marktüblichen Type I Glasrohr mit vorgegebener Toleranz abgelängt ist.

4. Behälter nach einem der Ansprüche 1 bis 3, **dadurch gekennzeichnet, daß** das Kunststoffgehäuse (10) mit angeformtem Kopfteil (9) ein Spritzgießteil ist.

5. Behälter nach Anspruch 4, **dadurch gekennzeichnet, daß** das Spritzgießteil transparent ist.

6. Behälter nach Anspruch 5, **dadurch gekennzeichnet, daß** das Spritzgießteil aus einem Cycloolefinen Polymer (COP) besteht.

7. Behälter nach Anspruch 5, **dadurch gekennzeichnet, daß** das Spritzgießteil aus einem Cycloolefinen Copolymer (COC) besteht.

8. Behälter nach Anspruch 5, **dadurch gekennzeichnet, daß** das Spritzgießteil aus einem Polycarbonat (PC) besteht.

9. Behälter nach Anspruch 5, **dadurch gekennzeichnet, daß** das Spritzgießteil aus einem Polystyrol (PS) besteht.

10. Behälter nach Anspruch 5, **dadurch gekennzeichnet, daß** das Spritzgießteil aus einem Acrylnitril-Butadien-Styrol-Propf-Polymer (ABS) besteht.

11. Behälter nach Anspruch 5, **dadurch gekennzeichnet, daß** das Spritzgießteil aus Polymethylmethacrylat (PMMA) besteht.

12. Behälter nach Anspruch 5, **dadurch gekennzeichnet, daß** das Spritzgießteil aus Polyethylenterephtalat (PET) besteht.
